# EUROPEAN PATENT APPLICATION

(11) **EP 3 586 764 A2**
(43) Date of publication of application: **01.01.2020**
(21) Application number: 19181512.5
(22) Date of filing: 20.06.2019
(51) Int. Cl.: A61B 17/02

(54) **SURGICAL RETRACTORS**

(30) Priority: 21.06.2018 US 201862687827 P; 26.03.2019 US 201916364779
(71) Applicant: Covidien LP, Mansfield, MA 02048 (US)
(72) Inventor: JADHAV, Amarsinh Deeliprao, 500050 Hyderabad (IN); MANKAR, Nikhil Prabhakar, 411057 Pune (IN); VIKHARANKAR, Yogesh Kishore, 500050 Hyderabad (IN)
(74) Representative: Maschio, Antonio

(57) **Abstract**

A surgical retractor includes an outer housing, an inner shaft, and a tool assembly. The outer housing defines a longitudinal axis and a longitudinal bore that extends along the longitudinal axis. The inner shaft is moveable within the outer housing. The tool assembly includes a shaft portion and a retractor member. The shaft portion is connected to the inner shaft by a pivot member that defines an axis that is perpendicular to the longitudinal axis of the outer housing. A distal portion of the shaft portion supports the retractor member, wherein the retractor member can pivot about the pivot member from a first position in which the shaft portion is aligned with the longitudinal axis of the outer housing to a second position in which the shaft portion is positioned at an acute angle with respect to the longitudinal axis of the outer housing.

## Description

### CROSS-REFERENCE TO RELATED APPLICATION

This application claims the benefit of and priority to U.S. Provisional Patent Application No. 62/687,827 filed June 21, 2018, the entire disclosure of which is incorporated by reference herein.

### BACKGROUND

### 1. Technical Description

The present disclosure is directed to surgical retractors, and more particularly, to surgical retractors with articulating tool assemblies.

### 2. Background of Related Art

Surgical retractors are commonly used during surgical procedures to displace internal organs to provide space within a body cavity to perform the surgical procedure. During certain surgical procedures, e.g., rectum dissection, colon resection, etc., the rectum/colon is mobilized or separated from surrounding tissues to render the rectum/colon accessible for resection. The procedure for mobilizing the rectum/colon is made difficult due to the position of intestines within the abdominal cavity, i.e., the intestines hang onto the rectum/colon to make access to the rectum/colon more difficult.

In order to provide access to the rectum/colon, the intestines are moved away from the rectum/colon using graspers. The graspers are inserted to the surgical site through ports in the abdominal wall and the intestines are gripped with the graspers and lifted. The use of graspers as described above to lift the intestines from the rectum/colon requires two ports and two hands to operate the graspers. As such, a procedure for mobilizing the rectum/colon requires a clinician and his assistant.

A continuing need exists in the art for a surgical device for lifting a body organ, such as the intestine, using a single hand and through a single port during an endoscopic procedure.

### SUMMARY

An aspect of the present disclosure is directed to a surgical retractor including an outer housing, an inner shaft, and a tool assembly. The outer housing defines a longitudinal axis and a longitudinal bore that extends along the longitudinal axis, and includes a proximal portion and a distal portion. The inner shaft is supported within the longitudinal bore of the outer housing and has a proximal portion and a distal portion. The inner shaft is moveable between a retracted position and an advanced position within the outer housing. The tool assembly includes a shaft portion and a retractor member. The shaft portion has a distal portion and a proximal portion. The proximal portion is pivotably connected to the distal portion of the inner shaft by a pivot member that defines an axis that is perpendicular to the longitudinal axis of the outer housing. The distal portion of the shaft portion supports the retractor member, wherein the retractor member can pivot about the pivot member from a first position in which the shaft portion is aligned with the longitudinal axis of the outer housing to a second position in which the shaft portion is positioned at an acute angle with respect to the longitudinal axis of the outer housing.

In some embodiments, the retractor member has a bowl-like configuration.

In certain embodiments, the retractor member defines a plurality of openings that prevents fluid from accumulating within the retractor member.

In embodiments, a handle is supported on the proximal portion of the outer housing and an actuator grip is supported on the proximal portion of the inner shaft.

In some embodiments, the retractor member includes a plurality of tines and a base member, wherein the plurality of tines extends distally from the base member.

In certain embodiments, each of the tines and the base member are curved along their longitudinal axes to define a slight concavity.

In embodiments, the surgical retractor includes a link having a proximal portion and a distal portion, wherein the distal portion is connected to the shaft portion of the tool assembly at a position laterally of the pivot member.

In some embodiments, the proximal portion of the link extends to a position adjacent the outer housing.

Another aspect of the present disclosure is directed to a method of performing a surgical procedure including inserting a surgical retractor through a cannula to position a distal portion of an outer housing of the surgical retractor adjacent a body organ; moving an inner shaft of the surgical retractor in relation to the outer housing of the surgical retractor to deploy a tool assembly of the surgical retractor from the outer housing; positioning the body organ on a retractor member of the tool assembly; and pivoting the retractor member in relation to the inner shaft about a pivot axis that is perpendicular to a longitudinal axis of the outer housing to lift the body organ.

In some embodiments, pivoting the retractor member in relation to the inner shaft includes pivoting a shaft portion of the tool assembly in relation to the inner shaft.

In certain embodiments, pivoting the retractor member in relation to the inner shaft includes retracting a link that includes a distal end that is coupled to the shaft portion of the tool assembly.

In embodiments, positioning the body organ on a retractor member of the tool assembly includes positioning the body organ within a concavity of the retractor member.

In some embodiments, positioning the body organ on a retractor member of the tool assembly includes positioning the body organ within a bowl of the retractor member.

In certain embodiments, positioning the body organ on a retractor member of the tool assembly includes positioning the body organ on tines of the retractor member.

In embodiments, the method also includes rotating the inner shaft within the outer housing to rotate the tool assembly about a longitudinal axis defined by the outer housing.

### BRIEF DESCRIPTION OF THE DRAWINGS

Various embodiments of the presently disclosed surgical retractor are described herein below with reference to the drawings, wherein:
FIG. 1 is a side perspective view of an exemplary embodiment of the presently disclosed surgical retractor in a deployed state;
FIG. 2 is a side perspective view of surgical retractor shown in FIG. 1 in a non-deployed state;
FIG. 3 is an exploded, perspective view of the surgical retractor shown in FIG. 2;
FIG. 4 is a cross-sectional view taken along section line 4-4 of FIG. 2;
FIG. 5 is an enlarged view of the indicated area of detail shown in FIG. 4;
FIG. 6 is a cross-sectional view taken along section line 6-6 of FIG. 2;
FIG. 7 is a cross-sectional view taken along section line 7-7 of FIG. 1;
FIG. 7A is an enlarged view of the indicated area of detail shown in FIG. 7;
FIG. 7B is side cross-sectional view of the surgical retractor shown in FIG. 7 in an articulated state;
FIG. 7C is an enlarged view of the indicated area of detail shown in FIG. 7B;
FIG. 8 is a cross-sectional view taken along section line 8-8 of FIG. 1;
FIG. 9 is a side perspective view of the surgical retractor shown in FIG. 8 in a deployed state as a retractor body of the surgical retractor is rotated in relation to a handle assembly of the surgical retractor;
FIG. 10 is a side perspective view of the surgical retractor shown in FIG. 9 extending through a cannula into a body cavity with the surgical retractor in a deployed state;
FIG. 11 is a side perspective view of another exemplary embodiment of the presently disclosed surgical retractor in a deployed state;
FIG. 12 is a side perspective view of surgical retractor shown in FIG. 11 in a non-deployed state;
FIG. 13 is a side perspective view of the surgical retractor shown in FIG. 12 in a deployed state as a retractor body of the surgical retractor is rotated in relation to a handle assembly of the surgical retractor;
FIG. 14 is an enlarged view of the retractor body of the surgical retractor shown in FIG. 13; and
FIG. 15 is a cross-sectional view taken along section line 15-15 of FIG. 12.

### DETAILED DESCRIPTION OF EMBODIMENTS

The presently disclosed surgical retractor will now be described in detail with reference to the drawings in which like reference numerals designate identical or corresponding elements in each of the several views. However, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

In this description, the term "proximal" is used generally to refer to that portion of the device that is closer to a clinician, while the term "distal" is used generally to refer to that portion of the device that is farther from the clinician. In addition, the term "endoscopic" is used generally used to refer to endoscopic, laparoscopic, arthroscopic, and/or any other procedure conducted through small diameter incision or cannula. Further, the term "clinician" is used generally to refer to medical personnel including doctors, nurses, and support personnel.

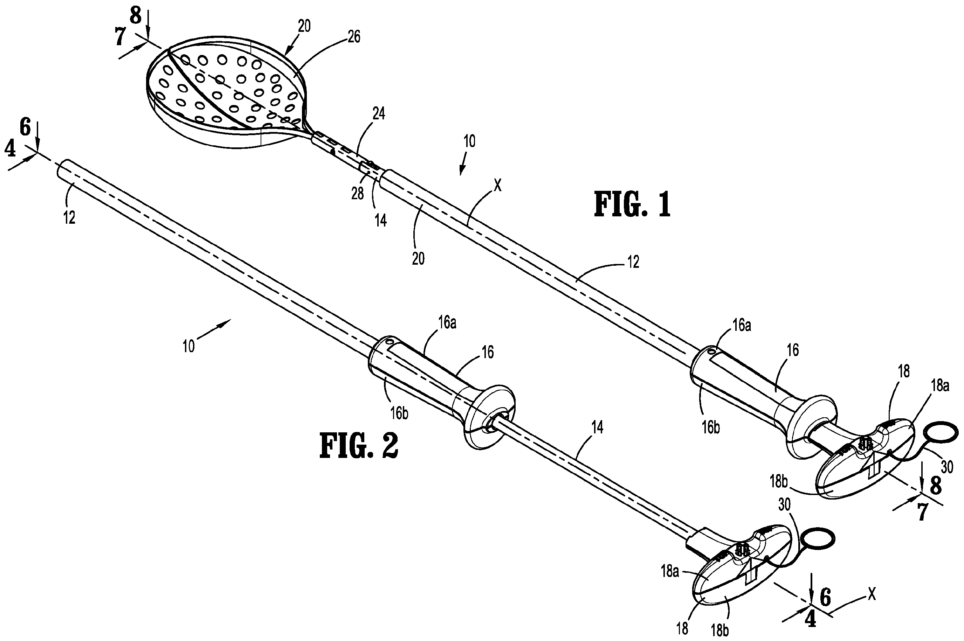

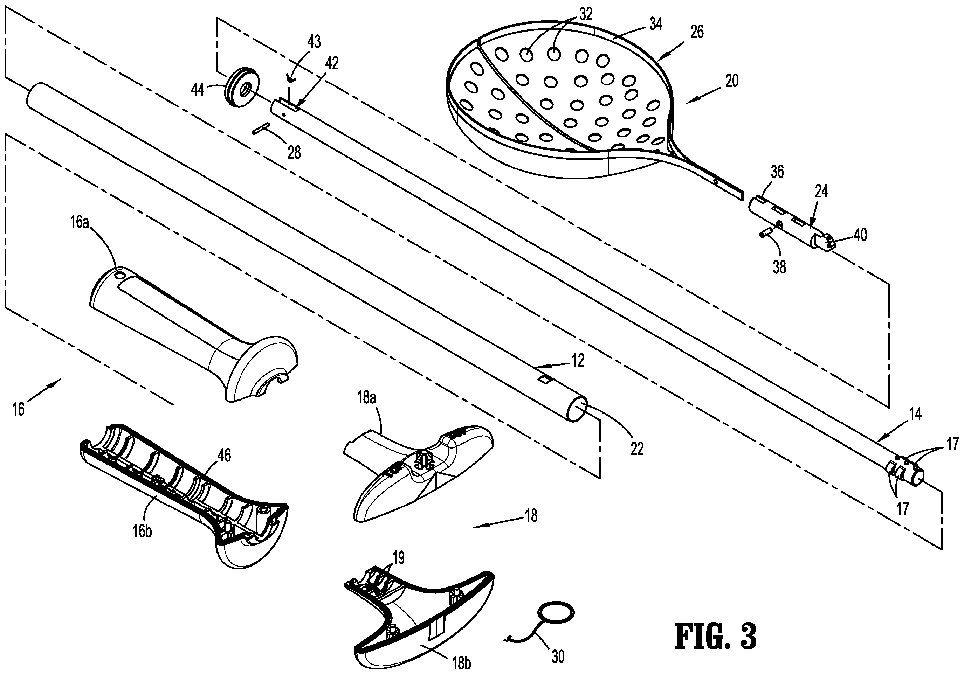

Referring to FIGS. 1-3, the presently disclosed surgical retractor is shown generally as retractor 10 and includes an outer housing 12 defining a longitudinal axis "X", an inner shaft 14, a handle 16, an actuator grip 18, and a tool assembly 20. The outer housing 12 is hollow and defines a longitudinal bore 22 (FIG. 3) that receives the inner shaft 14 such that the inner shaft 14 is slidable within the longitudinal bore 22 of the outer housing 12 between a retracted position (FIG. 2) and an advanced position (FIG. 1). The handle 16 is fixedly secured to a proximal portion of the outer housing 12. In embodiments, the handle 16 is formed from half-sections 16a, 16b that are secured together about the proximal portion of the outer shaft 12 using, e.g., screws. Similarly, the actuator grip 18 can also be formed of half-sections 18a, 18b that are secured about the proximal portion of inner shaft 14 using, e.g., screws. Alternately, other securing techniques can be used to secure the handle 16 to the outer housing 12 and/or the actuator grip 18 to the inner shaft 14. In embodiments, the proximal portion of the inner shaft 14 may include protrusions 17 (FIG. 3) that are received within slots 19 (FIG. 3) defined by the grip half-sections 18a, 18b to axially secure the actuator grip 18 to the inner shaft 14.

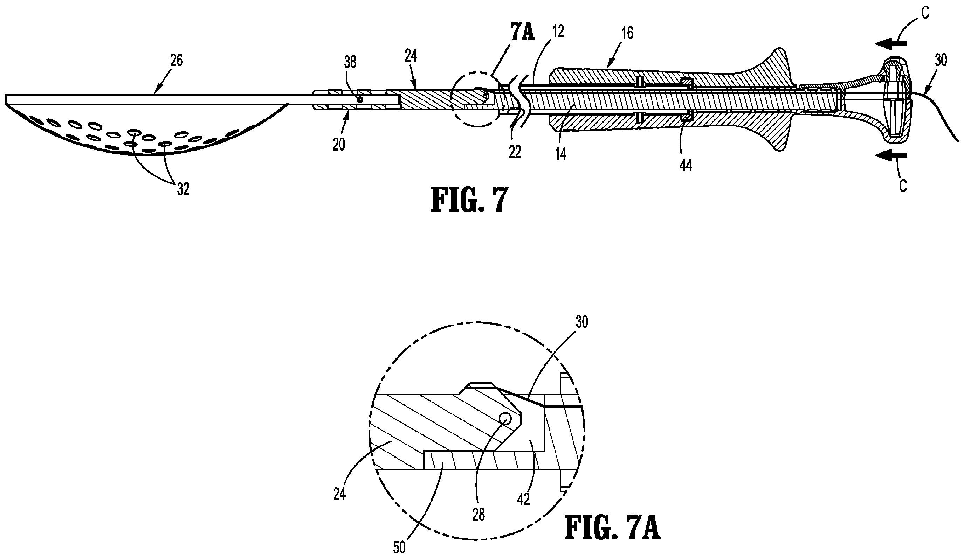

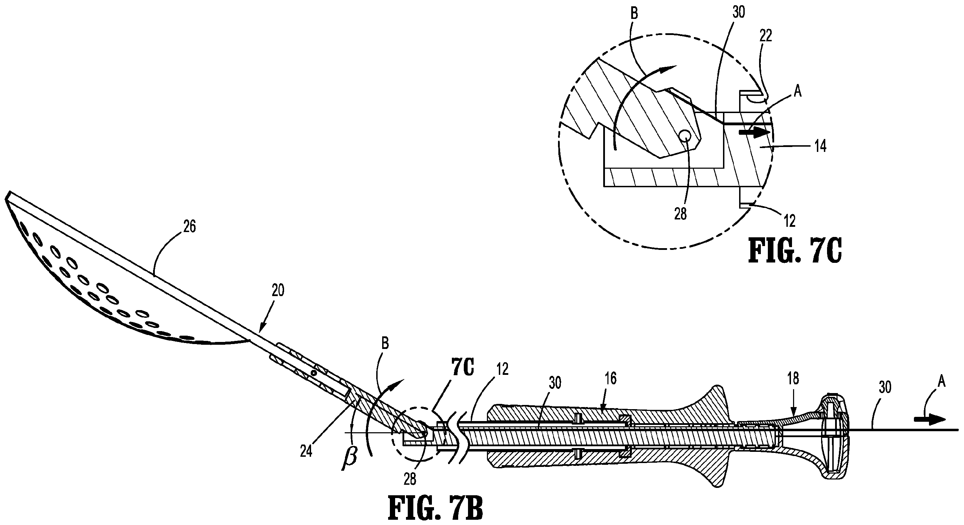

The tool assembly 20 includes a shaft portion 24 and a tissue retractor member 26. The shaft portion 24 includes a proximal shaft portion and a distal shaft portion. The distal portion of the shaft portion 24 supports the tissue retractor member 26 and the proximal portion of the shaft portion 24 is pivotally coupled to the inner shaft 14 about a pivot pin 28 that extends in a direction substantially perpendicular to the longitudinal axis "X" of the outer housing 12. As such, the tool assembly 20 can pivot between a position in which the shaft portion 24 is aligned with the longitudinal axis "X" and a position in which the shaft portion 24 forms an acute angle β (FIG. 7B) with the longitudinal axis "X".

In embodiments, the surgical retractor 10 includes a link 30 having a distal end that is coupled to the shaft portion 24 of the tool assembly 20 at a point that is laterally offset from the pivot pin 28 such that movement of the link 30 in the direction indicated by arrow "A" in FIG. 7C causes the tool assembly 20 to pivot about the pivot pin 28 in the direction indicted by arrow "B". A proximal end of the link 30 extends to a position adjacent the handle 16. As described in further detail below, the tool assembly 20 may be pivoted in the direction indicated by arrow "B" by actuating the link 30 to lift the intestine "I" during a procedure to mobilize the rectum/colon.

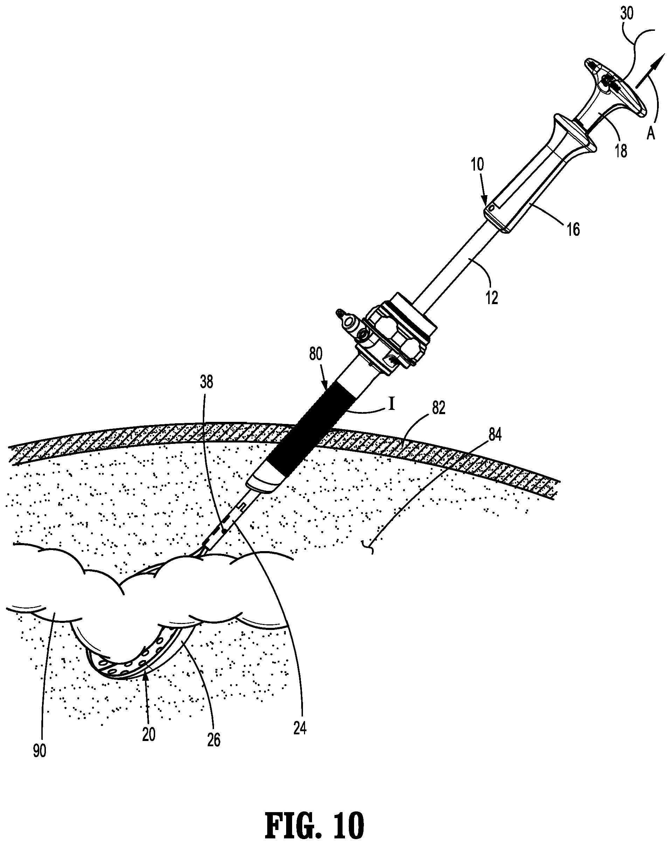

Referring to FIGS. 3-6, when the inner shaft 14 is in a retracted position, the tool assembly 20 including the shaft portion 24 and the retractor member 26 is positioned within the longitudinal bore 22. In embodiments, the retractor member 26 is formed of a resilient material, e.g., spring steel, and can be furled or folded to a configuration to be received within the longitudinal bore 22 of the outer housing 12. In some embodiments, the retractor member 26 has a collapsible bowl-like configuration that defines a concavity that is configured to receive and support a body organ, e.g., intestine "I" (FIG. 10) during a surgical procedure. In certain embodiments, the retractor member 26 defines one or more openings 32 (FIG. 3) that allow fluid to flow through the retractor member 26 so that fluid does not collect in the retractor member 26.

Referring to FIG. 3, in embodiments, the retractor member 26 of the tool assembly 20 includes a resilient band 34 (FIG. 3) that is received within a slot 36 formed in a distal end of the shaft portion 24 of the tool assembly 20 and secured to the shaft portion 24 with a pin 38. Alternately, the retractor member 26 can be secured to the shaft portion 24 using a variety of different fastening techniques.

The proximal portion of the shaft portion 24 of the tool assembly 20 includes an extension 40 that is received within a clevis 42 formed on the distal portion of the inner shaft 14. The extension 40 is coupled to the clevis 42 by the pivot pin 28 to secure the shaft portion 24 of the tool assembly 20 to the inner shaft 14. In the retracted position, the shaft portion 24 is positioned within the outer housing 12 and cannot pivot. A spring or biasing member 43 may be provided to urge the tool assembly 20 to a non-articulated position (FIG. 7) in relation to the inner shaft 14.

In some embodiments, a ring seal 44 is supported within a slot 46 (FIG. 3) defined between the half-sections 16a, 16b of the handle 16. The ring seal 44 is received about the inner shaft 14 and prevents fluid flow between the inner shaft 14 and the outer housing 12 through the longitudinal bore 22.

Referring to FIGS. 7 and 7A, when the inner shaft 14 is moved in relation to outer housing 12 in the direction of arrows "C" towards the advanced position, the tool assembly 20 is pushed from the longitudinal bore 22 of the outer housing 12. When tool assembly 20 including the retractor member 26 exits the longitudinal bore 22 of the outer housing 12, the retractor member 26 returns to an unbiased, deployed state having a bowl-like configuration. When the inner shaft 14 is in the advanced position, the clevis 42 is positioned externally of the longitudinal bore 22 of the outer housing 12 such that the tool assembly 20 can be pivoted in relation to the inner shaft 14 and outer housing 12.

As shown in FIG. 7A, the clevis 42 may be configured to facilitate articulation of the tool assembly 20 in only one direction. For example, as shown in FIG. 7A, the distal end of the inner shaft 14 may include a stop member 50 that prevents articulation of the tool assembly 20 in a downward direction as viewed in FIG. 7.

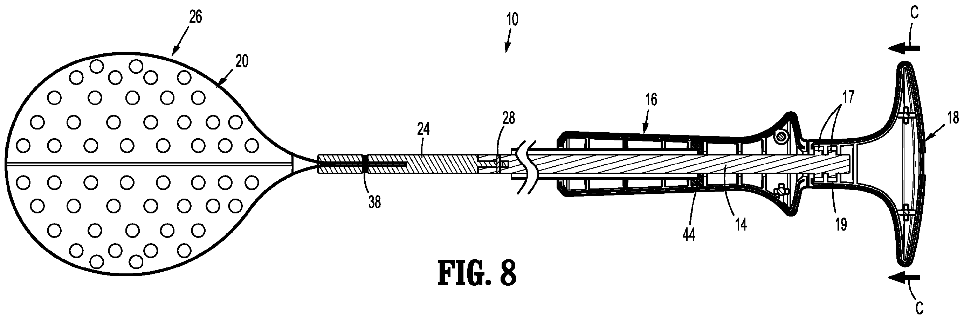

Referring to FIGS. 7B-8, in order to articulate the tool assembly 20 about the pivot pin 28, the link 30 can be pulled in the direction indicated by arrows "A" in FIG. 7B. Since the distal portion of the link 30 is connected to the proximal portion of the shaft portion 24 of the tool assembly 20 at a position offset from the pivot pin 28, movement of the link 30 in the direction "A" will pivot the tool assembly 20 about the axis defined the pivot pin 28 in the direction indicated by arrow "B" in FIG. 7B. Although not shown, a proximal end of the link 30 may include a hand grip to facilitate grasping of the link 30 by the clinician.

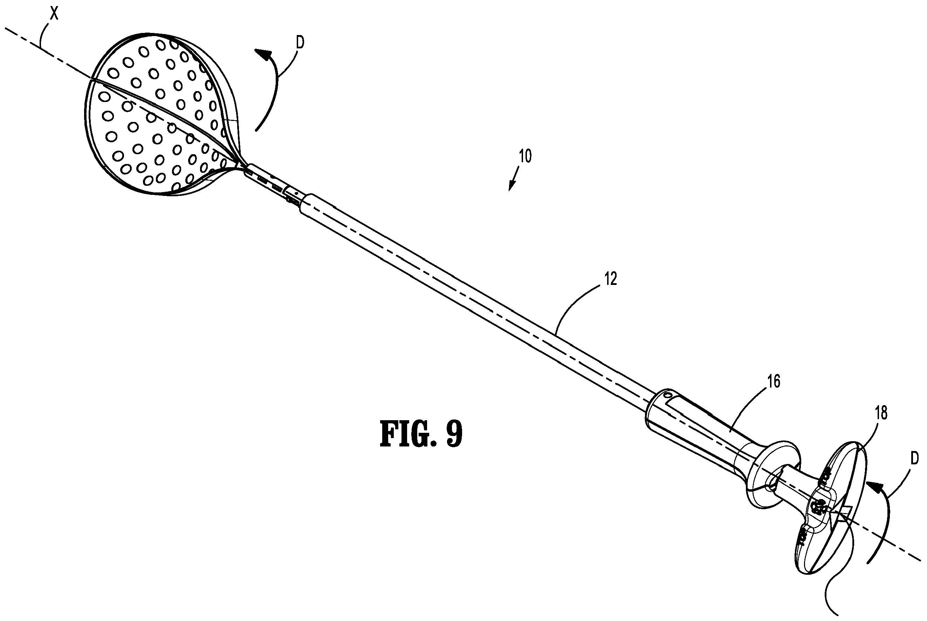

Referring to FIG. 9, in embodiments, the inner shaft 14 can be rotated within the outer housing 12 about the longitudinal axis "X" in the direction indicated by arrows "D". This can be accomplished by holding the handle 16 stationary and rotating the actuator grip 18 in the direction indicated by arrow "D" or in the opposite direction.

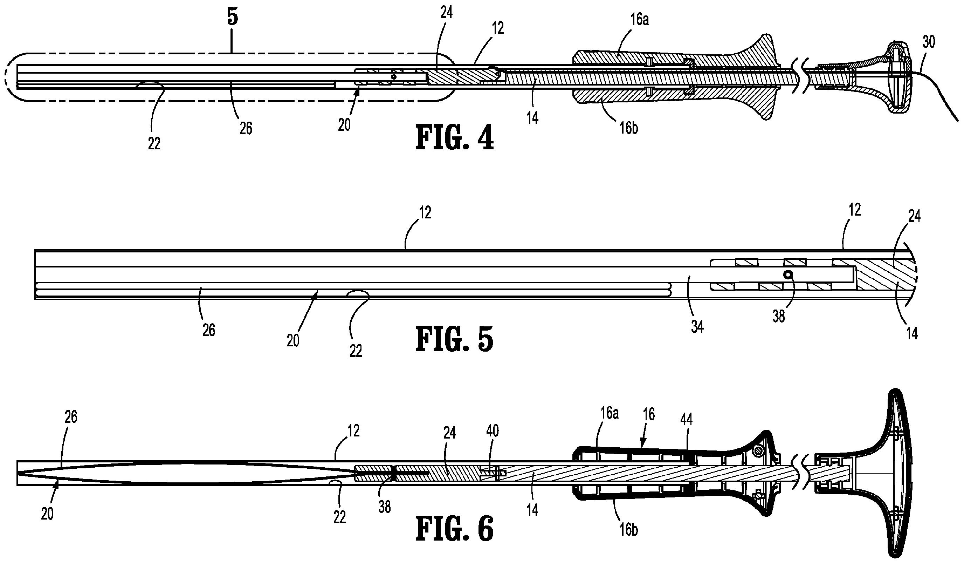

Referring to FIG. 10, during an endoscopic surgical procedure such as a procedure to immobilize the rectum/colon, the outer housing 12 of the surgical retractor 10 is inserted through a cannula 80 that extends through an incision "I" in the abdominal wall 82 to access the abdominal cavity 84. During insertion of the outer housing 12 through the cannula 80, the inner shaft 14 is in a retracted position with the tool assembly 20 confined within the longitudinal bore 22 (FIG. 6) of the outer housing 12. Once the distal end of the outer housing 12 is positioned adjacent tissue to be displaced with the surgical retractor 10, e.g., the intestines 90, the inner shaft 14 can be moved to the advanced position within the outer housing 12 to move the surgical retractor 10 to the deployed state with the tool assembly 20 extending from the outer housing 12. At this point, the retractor member 26 of the tool assembly 20 can be manipulated to lift the intestine "I" upwardly to move the intestines "I" off of the rectum/colon to provide access to the rectum/colon.

In some embodiments, in order to further lift the intestine "I" off of the rectum/colon, the link 30 can be pulled proximally (FIG. 7A) to pivot the tool assembly 20 about the pivot member 28. After the surgical procedure is completed, the inner shaft 12 can be retracted by pulling the actuator grip 18 proximally while holding the handle 16 stationary to move the tool assembly 20 back to the non-deployed state positioned within the outer tube 12.

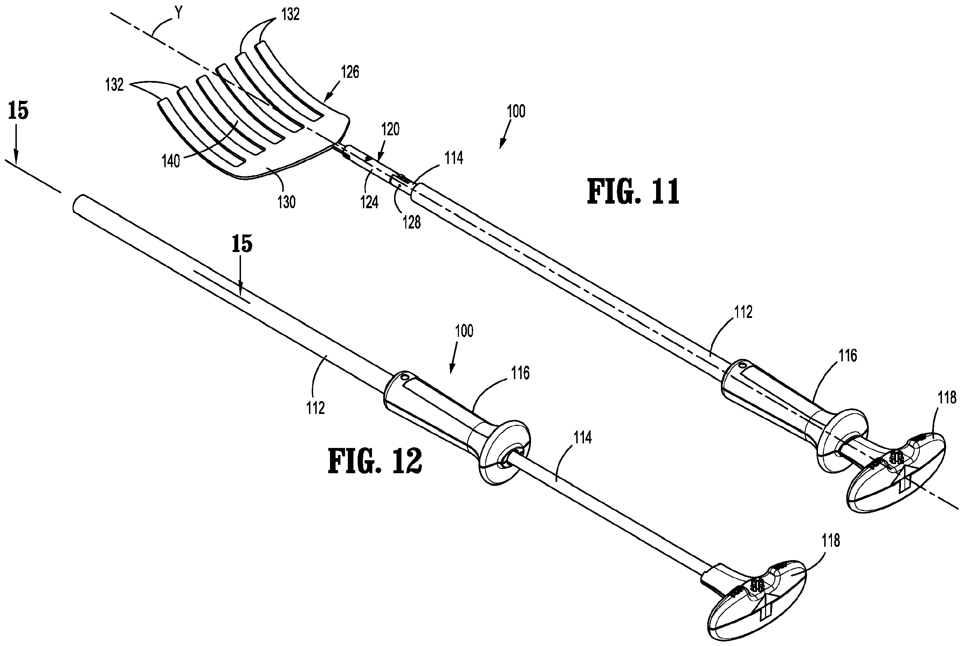

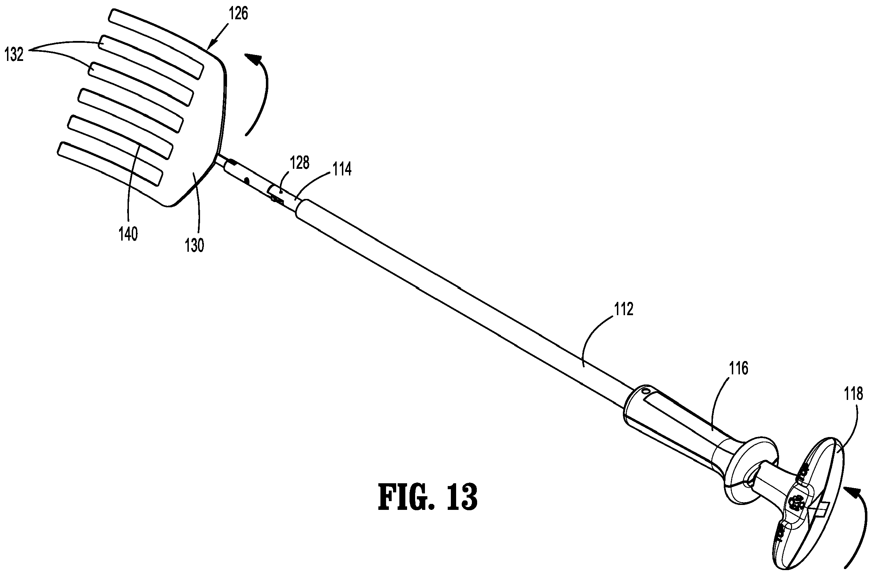

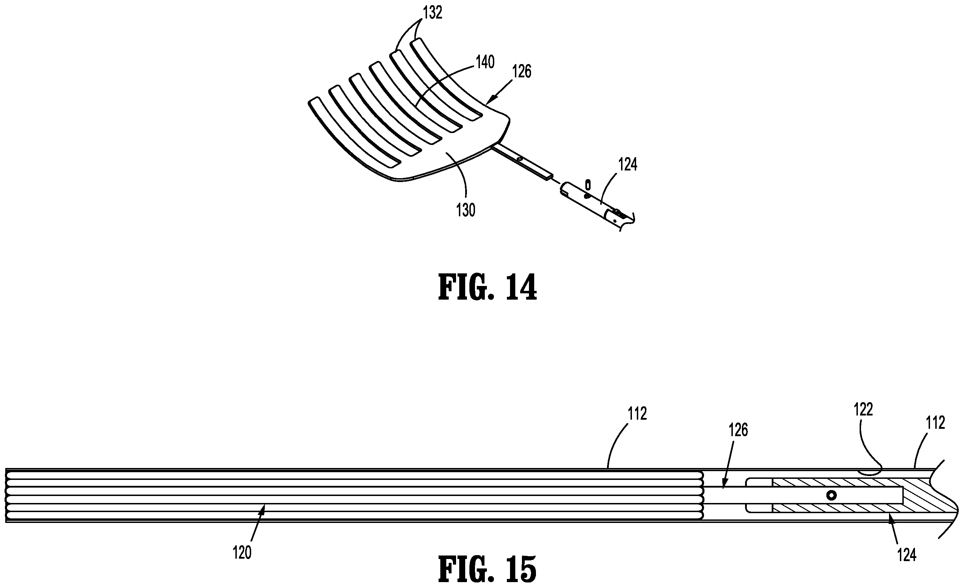

FIGS. 11-15 illustrate another exemplary embodiment of the presently disclosed surgical retractor shown generally as retractor 100. The surgical retractor 100 includes an outer housing 112 defining a longitudinal axis "Y", an inner shaft 114, a handle 116, an actuator grip 118, and a tool assembly 120. The tool assembly 120 includes a shaft portion 124 and a retractor member 126. The outer housing 112 is hollow and defines a longitudinal bore 122 (FIG. 15). The longitudinal bore 122 is dimensioned to receive the inner shaft 114 such that the inner shaft 114 is slidable within the longitudinal bore 122 between a retracted position (FIG. 12) and an advanced position (FIG. 11). The handle 116 is fixedly secured to a proximal portion of the outer housing 112. The distal portion of the inner shaft 114 supports the tool assembly 120. The surgical retractor 100 is substantially as described above with regard to the surgical retractor 10 except that the configuration of the retractor member 126 is changed. As such, only the retractor member 126 of the surgical retractor 100 will be described in further detail herein.

The shaft portion 124 is pivotably coupled to the retractor member 126 by a pivot pin 128 in substantially the same manner that the shaft portion 24 is pivotably coupled to the retractor member 26. As such, no further description of the pivot structure of the surgical retractor 100 is provided herein. The retractor member 126 includes a base portion 130 and a series of tines 132 that extend distally from the base portion 132. In embodiments, the base portion 132 and the tines 130 are curved along their longitudinal axis "Y" such that the retractor member 126 defines a slight concavity140. The surgical retractor 100 operates in substantially the same manner as the surgical retractor 10. Accordingly, no further description of the is provided herein.

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described embodiments. Accordingly, the disclosure is not to be limited by what has been particularly shown and described, except as indicated by the appended claims.

The invention may be described by reference to the following numbered paragraphs:-
1. A surgical retractor comprising:
   an outer housing defining a longitudinal axis and a longitudinal bore, the outer housing having a proximal portion and a distal portion;
   an inner shaft supported within the longitudinal bore of the outer housing and having a proximal portion and a distal portion, the inner shaft being moveable between a retracted position and an advanced position within the outer housing;
   a tool assembly including a shaft portion and a retractor member, the shaft portion having a distal portion and a proximal portion, the proximal portion being pivotably connected to the distal portion of the inner shaft by a pivot member, the pivot member defining an axis that is perpendicular to the longitudinal axis of the outer housing, the distal portion of the shaft portion supporting the retractor member, wherein the retractor member can pivot about the pivot member from a first position in which the shaft portion is aligned with the longitudinal axis of the outer housing to a second position in which the shaft portion is positioned at an acute angle with respect to the longitudinal axis of the outer housing.
2. The surgical retractor of paragraph 1, wherein the retractor member has a bowl-like configuration.
3. The surgical retractor of paragraph 2, wherein the retractor member defines a plurality of openings that prevents fluid from accumulating within the retractor member.
4. The surgical retractor of paragraph 2, further including a handle supported on the proximal portion of the outer housing and an actuator grip supported on the proximal portion of the inner shaft.
5. The surgical retractor of paragraph 1, wherein the retractor member includes a plurality of tines and a base member, the plurality of tines extending distally from the base member.
6. The surgical retractor of paragraph 5, wherein each of the tines and the base member are curved along their longitudinal axes to define a slight concavity.
7. The surgical retractor of paragraph 1, further including a link having a proximal portion and a distal portion, the distal portion being connected to the shaft portion of the tool assembly at a position laterally of the pivot member.
8. The surgical retractor of paragraph 7, wherein the proximal portion of the link extends to a position adjacent the outer housing.
9. A method of performing a surgical procedure comprising;
   inserting a surgical retractor through a cannula to position a distal portion of an outer housing of the surgical retractor adjacent a body organ;
   moving an inner shaft of the surgical retractor in relation to the outer housing of the surgical retractor to deploy a tool assembly of the surgical retractor from the outer housing;
   positioning the body organ on a retractor member of the tool assembly; and
   pivoting the retractor member in relation to the inner shaft about a pivot axis that is perpendicular to a longitudinal axis of the outer housing to lift the body organ.
10. The method of paragraph 9, wherein pivoting the retractor member in relation to the inner shaft includes pivoting a shaft portion of the tool assembly in relation to the inner shaft.
11. The method of paragraph 10, wherein pivoting the retractor member in relation to the inner shaft includes retracting a link that includes a distal end that is coupled to the shaft portion of the tool assembly.
12. The method of paragraph 9, wherein positioning the body organ on a retractor member of the tool assembly includes positioning the body organ within a concavity of the retractor member.
13. The method of paragraph 12, wherein positioning the body organ on a retractor member of the tool assembly includes positioning the body organ within a bowl of the retractor member.
14. The method of paragraph 12, wherein positioning the body organ on a retractor member of the tool assembly includes positioning the body organ on tines of the retractor member.
15. The method of paragraph 9, further including rotating the inner shaft within the outer housing to rotate the tool assembly about a longitudinal axis defined by the outer housing.

## Claims

1. A surgical retractor comprising:
an outer housing defining a longitudinal axis and a longitudinal bore, the outer housing having a proximal portion and a distal portion;
an inner shaft supported within the longitudinal bore of the outer housing and having a proximal portion and a distal portion, the inner shaft being moveable between a retracted position and an advanced position within the outer housing;
a tool assembly including a shaft portion and a retractor member, the shaft portion having a distal portion and a proximal portion, the proximal portion being pivotably connected to the distal portion of the inner shaft by a pivot member, the pivot member defining an axis that is perpendicular to the longitudinal axis of the outer housing, the distal portion of the shaft portion supporting the retractor member, wherein the retractor member can pivot about the pivot member from a first position in which the shaft portion is aligned with the longitudinal axis of the outer housing to a second position in which the shaft portion is positioned at an acute angle with respect to the longitudinal axis of the outer housing.

2. The surgical retractor of claim 1, wherein the retractor member has a bowl-like configuration.

3. The surgical retractor of claim 2, wherein the retractor member defines a plurality of openings that prevents fluid from accumulating within the retractor member.

4. The surgical retractor of claim 2 or claim 3, further including a handle supported on the proximal portion of the outer housing and an actuator grip supported on the proximal portion of the inner shaft.

5. The surgical retractor of any preceding claim, wherein the retractor member includes a plurality of tines and a base member, the plurality of tines extending distally from the base member.

6. The surgical retractor of claim 5, wherein each of the tines and the base member are curved along their longitudinal axes to define a slight concavity.

7. The surgical retractor of any preceding claim, further including a link having a proximal portion and a distal portion, the distal portion being connected to the shaft portion of the tool assembly at a position laterally of the pivot member.

8. The surgical retractor of claim 7, wherein the proximal portion of the link extends to a position adjacent the outer housing.

9. A method of performing a surgical procedure comprising;
inserting a surgical retractor through a cannula to position a distal portion of an outer housing of the surgical retractor adjacent a body organ;
moving an inner shaft of the surgical retractor in relation to the outer housing of the surgical retractor to deploy a tool assembly of the surgical retractor from the outer housing;
positioning the body organ on a retractor member of the tool assembly; and
pivoting the retractor member in relation to the inner shaft about a pivot axis that is perpendicular to a longitudinal axis of the outer housing to lift the body organ.

10. The method of claim 9, wherein pivoting the retractor member in relation to the inner shaft includes pivoting a shaft portion of the tool assembly in relation to the inner shaft.

11. The method of claim 10, wherein pivoting the retractor member in relation to the inner shaft includes retracting a link that includes a distal end that is coupled to the shaft portion of the tool assembly.

12. The method of any of claims 9 to 11, wherein positioning the body organ on a retractor member of the tool assembly includes positioning the body organ within a concavity of the retractor member.

13. The method of claim 12, wherein positioning the body organ on a retractor member of the tool assembly includes positioning the body organ within a bowl of the retractor member.

14. The method of any of claims 9 to 13, wherein positioning the body organ on a retractor member of the tool assembly includes positioning the body organ on tines of the retractor member.

15. The method of any of claims 9 to 14, further including rotating the inner shaft within the outer housing to rotate the tool assembly about a longitudinal axis defined by the outer housing.
